**Europäisches Patentamt**

**European Patent Office**

**Office européen des brevets**

(19)

(11) **EP 1 172 069 A1**

(12) **EUROPÄISCHE PATENTANMELDUNG**

(43) Veröffentlichungstag:
**16.01.2002 Patentblatt 2002/03**

(51) Int Cl.⁷: $A61B\ 6/03$, $G01N\ 23/04$

(21) Anmeldenummer: **00115267.7**

(22) Anmeldetag: **14.07.2000**

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(71) Anmelder: **VAMP Verfahren und Apparate der Medizinischen Physik GmbH**
**91096 Möhrendorf (DE)**

(72) Erfinder:
- **Kalender, Willi A., Dr.**
  **91096 Kleinseebach (DE)**
- **Kachelriess, Marc, Dr.**
  **90409 Nürnberg (DE)**

(54) **Computertomograph mit Dosisoptimierung durch Festlegung der optimalen Wahl des Röhrenstroms in Echtzeit (Belichtungsautomatik), der Röhrenstrommodulation (Dosisminimierung) und darauf aufbauender Nachverarbeitung durch 3D adaptive Filter (Rauschreduzierung**

(57)   Die vorliegende Erfindung beschreibt einen Computertomographen mit Spiralabtastung (Ein- oder Mehrzeilendetektor), der während der Messung aus den Aufnahmedaten Informationen über das zu erwartende Bildpunktrauschen errechnet und den Röhrenstrom so regelt, dass das Bildpunktrauschen einen vorbestimmten Wert unabhängig vom gemessenen Objekt annimmt (Belichtungsautomatik) und dass die Dosis minimiert wird (Röhrenstrommodulation). Außerdem wird beschrieben wie die sogenannte 3D adaptive Filterung modifiziert werden kann, damit sie optimalen Nutzen aus dem so gewonnenen zeitlichen Verlauf des Röhrenstroms zieht.

EP 1 172 069 A1

# EP 1 172 069 A1

**Beschreibung**

Die moderne Röntgen-Computertomographie (CT) hat durch neue Scanverfahren und durch Verbesserungen der Technologie ein breites Anwendungsspektrum erreicht. Die Spiral-CT, kontinuierliche Datenakquisition bei gleichzeitigem Patientenvorschub, ermöglicht sehr schnelle Volumenaufnahmen [1,2]. Um die damit möglichen Volumenaufnahmen weiter zu beschleunigen, wurden zwischenzeitlich Scanner mit mehr als 1 Detektorzeile entwickelt, die weniger als 1 Sekunde pro Umdrehung für die Datenerfassung benötigen. Durch die Möglichkeit, immer größere Volumina mit immer höherer Ortsauflösung zu scannen, steigen die Anforderungen an die Röntgenleistung und auch die mittlere Patientendosis pro Scan.

Die Patientendosis bei CT-Untersuchungen hat aus strahlenschutzrechtlichen, ethischen und ökonomischen Überlegungen sehr hohe Bedeutung. Es ist eine allgemeine Forderung, dass eine definierte Bildqualität mit niedrigst möglicher Dosis erzielt werden sollte. Es gibt aber für die CT keine Ansätze oder Vorschriften, wie dies in der Praxis im Detail durchzuführen ist. Eine Belichtungsautomatik, wie in der konventionellen Radiographie seit langem bekannt, gibt es für die CT noch nicht. Basierend auf früheren Untersuchungen und Arbeiten zur Röhrenstrommodulation [3,4,5], deren technische Verfügbarkeit eine Grundvoraussetzung für diese Patentanmeldung ist, schlagen wir ein Verfahren vor, wie das niedrigst mögliche mAs-Produkt und die Modulation der mA während eines Umlaufs ständig an die sich ändernden Objektquerschnitte und Schwächungsverhältnisse angepasst wird.

Primäres Ziel ist, einen vorgegebenen Wert des Bildpunktrauschens (Pixelrauschen) und eine möglichst homogene Rauschverteilung im rekonstruierten Volumendatensatz selbst bei stark variierenden Objektquerschnitten (z. B. Übergang von Schulter zu Hals) zu erreichen. Damit wäre die erzielte Bildqualität erstmals unabhängig vom untersuchten Objekt und störende Bildqualitätsschwankungen abhängig vom Patientenquerschnitt (zu hohes Bildrauschen bei dickeren Patienten und zu hohe Dosis bei dünneren Patienten) würden automatisch vermieden werden.

Die grundlegende Idee besteht darin, schritthaltend mit der Datenakquisition Rechnungen zur Höhe des zu erwartenden Rauschpegels durchzuführen und dementsprechend den mittleren Röhrenstrom (mA-Wert) so zu regeln, dass Veränderungen im Rauschpegel kompensiert werden.

Da die Modulation des Röhrenstroms nur komplette Projektionen und nicht gezielt einzelne Messwerte innerhalb einer Projektion beinflussen kann, verspricht eine Nachbearbeitung der gemessenen Daten durch geeignete softwarebasierte Algorithmen (eventuell in Form eines Vorverarbeitungsschrittes bei der Rekonstruktion) zusätzliche Verbesserung der Dosisnutzung und somit der Bildqualität. Außerdem können dadurch Einschränkungen in der Wahl des Röhrenstroms (z.B. Trägheit der Modulation) korrigiert werden. Stand der Technik sind so genannte adaptive Filter, die eine lokale Glättung nur einiger weniger, geschickt ausgewählter Projektionswerte in bis zu drei Dimensionen (Detektorrichtung, z-Richtung sowie zwischen einzelnen Projektionen) durchführen [6,7]. Damit werden bereits beachtliche Erfolge erzielt, jedoch werden bisher Schwankungen im Röhrenstrom nicht berücksichtigt. Eine Kombination der 3D adaptiven Filterung mit der Röhrenstrommodulation erscheint deshalb äußerst nutzbringend.

Zusammenfassend lassen sich folgende Ziele festhalten:

1. Ein Spiral-Computertomograph (Einzeiler, Mehrzeiler oder Kegelstrahlscanner), der während der Messung Informationen über das lokal zu erwartende Pixelrauschen berechnet und den Röhrenstrom entsprechend regelt, um das gewünschte Pixelrauschen zu erreichen.
(→Patentanspruch 1,2,3,4,5)
2. Eine modifizierte Methode zur Röhrenstrommodulation, die, aufsetzend auf die o.g. Röhrenstromregelung, lokal (während eines Umlaufs) den Röhrenstrom derart anpasst, dass die Rauschstrukturen im rekonstruierten Bild minimiert werden.
(→Patentanspruch 6)
3. Eine Möglichkeit zur dreidimensionalen adaptiven Filterung, die die Variation des Röhrenstroms berücksichtigt. Damit können die Ergebnisse der hardwarebasierten Röhrenstrommodulation verbessert und Mängel in der Röhrenstromregelung (z.B. Trägheit) softwaremäßig reduziert werden.
(→Patentanspruch 7)

**Lösung**

Der in Patentanspruch 1 angegebenen Erfindung liegt die Aufgabe zugrunde, für einen Spiral-Computertomographen eine Belichtungsautomatik mit angepasster Röhrenstrommodulation sowie Nachverarbeitungsmöglichkeit durch 3D adaptive Filter zu schaffen.

Die Aufgabe ist erfindungsgemäß gelöst durch die Merkmale der Patentansprüche.

Die Erfindung ist nachfolgend erläutert. Dabei wird als Beispiel jeweils auf medizinische Anwendungen Bezug genommen, ohne dass damit die Allgemeingültigkeit der Aussagen eingeschränkt wird.

Bezeichnungen:

Die Lösungsvorschläge beziehen sich o.B.d.A. (ohne Beschränkung der Allgemeinheit) auf den heute in der Medizin typischen Fall eines Spiral-Computertomographen in Fächergeometrie dessen Detektor zylindrisch und auf den Röntgenfokus zentriert ist. Die aus den Messungen gewonnenen Fächerdaten (Schwächungswerte) können im Laufe der Rekonstruktion durch ein Rebinning genanntes Umsortierungsverfahren auf Parallelgeometrie gebracht werden um die weitere Verarbeitung (Rekonstruktion) zu vereinfachen. Die beispielhafte Verwendung dieser Methoden (zylindrischer Detektor, Parallelrebinning etc.) schränkt die allgemeine Anwendbarkeit des Verfahrens und damit die vorgeschlagene Erfindung nicht ein. Denkbar sind unter Anderem der Einsatz in Kegelstrahl-Computertomographiegeräten, die oft einen planaren Detektor besitzen.

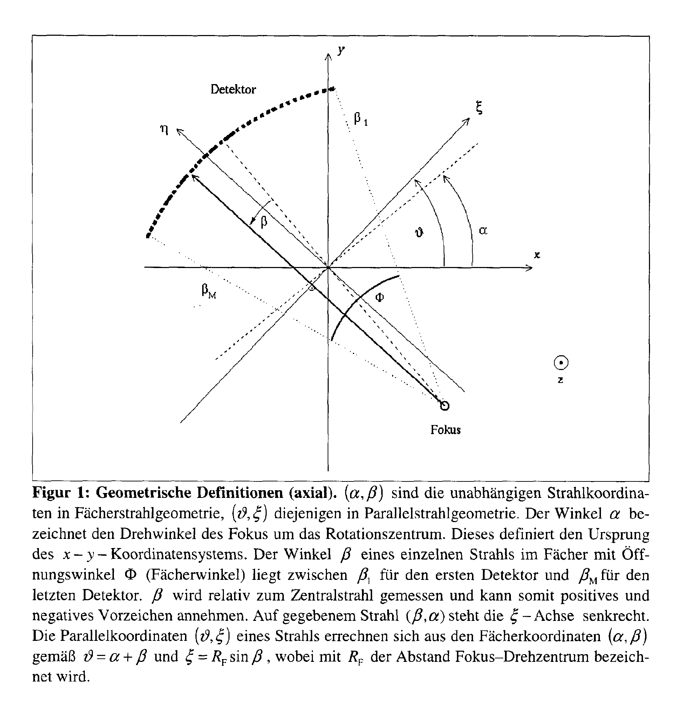

Es bezeichne $p(\alpha,\beta,m)$ die gemessenen Spiralrohdaten (Schwächungswerte). Dabei sei $\beta$ der Winkel im Fächer, $\alpha$ der Projektionswinkel und $m$ die Nummer der Detektorzeile (vgl. Abbildung 1). Die z-Position des Fokus hängt mit

$$z = d\alpha/2\pi$$

eindeutig vom Projektionswinkel $\alpha$ ab und tritt deshalb nicht als explizite Variable auf; $d$ sei der Tischvorschub pro 360° Gantryrotation. Die z-Position der Detektorzeile $m$ errechnet sich aus der kollimierten Schichtdicke S (auf das Rotationszentrum umgerechnete Kollimierung der Detektorzeilne) und der Fokusposition zu $z(\alpha, m) = z(\alpha) + S \times (m - (M + 1)/2)$. $M$ bezeichnet hier die Gesamtanzahl der Detektorzeilen, und es wurde o.B.d.A. angenommen, dass das Detektorarray in z-Richtung auf die Fokusposition zentriert sei.

Im Folgenden werden mögliche Lösungsbeispiele detailliert erläutert. Zufallsvariable werden mit Großbuchstaben bezeichnet und eine zugehörige Realisierung (das ist die Messung) mit Kleinbuchstaben. So bezeichnen beispielsweise $P_{\vartheta,\xi}$ und $p(\vartheta,\xi)$ die Zufallsvariable und die zugehörige Realisierung des Messwerts eines Strahls mit Abstand $\xi$ vom Ursprung und Winkel $\vartheta$ zu den Koordinatenachsen (s. Abb. 1).

**Lösung Ziel 1: On-line Bestimmung des Bildpunkrauschens (Belichtungsautomatik)**

Methode 1, direkte Verwendung der Rohdaten:

Die direkte Verwendung der Rohdaten basiert auf der Tatsache, dass die Varianz der gemessenen Schwächungswerte relativ gut durch die Beziehung

$$\mathrm{Var}P_{\vartheta,\xi} \approx aI^{-1}e^{P_{\vartheta,\xi}}$$

abgeschätzt werden kann, wobei $a$ eine Proportionalitätskonstante ist, die durch Kalibriermessungen oder auch durch direkte Analyse der Rohdaten leicht bestimmt werden kann und I die Primärintensität (= Anzahl der Quanten die bei Nichtvorhandenseins eines Objektes, d.h. bei einer Luftmessung, den Detektor $(\vartheta,\xi)$ treffen) oder der dazu proportionale Röhrenstrom ist. Da die Bildrekonstruktion ein linearer Prozess ist, kann durch Fehlerfortpflanzung das Rauschen im Bildraum einfach bestimmt werden. Angenommen, ein Bild entsteht durch gefilterte Rückprojektion (Faltungskern $k(\xi)$), d.h.

$$f(x,y) = \int d\vartheta\, d\xi\; p(\vartheta,\xi)k(x\cos\vartheta+y\sin\vartheta-\xi),$$

dann erhält man das zugehörige Varianzbild $\sigma^2(x, y)$ durch gefilterte Rückprojektion der Varianzen der Messwerte unter Verwendung des quadrierten Kerns:

$$\sigma^2(x, y) = a \int d\vartheta\, d\xi\; I^{-1}e^{p(\vartheta,\xi)}\, k^2(x\cos\vartheta+y\sin\vartheta-\xi)$$

Diese Feststellung alleine genügt noch nicht, um den Rechenaufwand im Vergleich zu einer kompletten Bildrekonstruktion zu reduzieren. Allerdings ist es zur Vorhersage des Rauschwertes nicht nötig, die komplette räumliche Rauschverteilung $\sigma^2(x, y)$ zu kennen sondern es reicht eine Art mittlere Varianz vorherzusagen. Diese soll in einer um den Ursprung (= Rotationszentrum) zentrierten ROI (Region of Interest) berechnet werden. In voller Allgemeinheit entspricht dies der mit einer radialen Funktion $w(r)$ gewichteten Aufintegration der Rauschverteilung $\sigma^2(x,y)$:

$$\sigma^2_{\text{mean}} = \int r\, dr\, d\varphi\, w(r)\sigma^2(r\cos\varphi,\, r\sin\varphi).$$

Dieses Integral kann vereinfacht werden zu

$$\sigma^2_{\text{mean}} = a \int d\xi d\vartheta\, e^{p(\vartheta,\xi)} k^2_{\text{mean}}(\xi)$$

mit

$$k^2_{mean}(\xi)= \int r\, dr\, d\varphi\, w(r)k^2(r\cos\varphi - \xi).$$

Damit besteht die Rauschvorhersage nur noch in einer zeilenweisen Aufintegration der relativen Intensitäten (Integral nach $d\vartheta$) mit anschließender gewichteter spaltenweisen Integration (Integral über $\xi$). Die Gewichtungsfunktion $k^2_{\text{mean}}(\xi)$ kann, wie in obiger Gleichung verdeutlicht, vorab berechnet und abgespeichert werden. Die Rauschvorhersage kann mit geringem Rechenaufwand realisiert werden, und die ist um mehrere Größenordnungen schneller als der Umweg über ein rekonstruiertes Bild.

Die Schwächungsdaten werden allerdings in Fächerstrahlgeometrie (Parameter $\alpha$ und $\beta$) und nicht in Parallelstrahlgeometrie (Parameter $\vartheta$ und $\xi$) gemessen (s. auch Abb. 1). Die notwendige Transformation

$$\xi = -R_{\text{F}}\sin\beta$$

$$\vartheta = \alpha + \beta$$

zum Übergang von Fächerstrahldaten $p(\alpha,\beta)$ auf Parallelstrahldaten $p(\vartheta,\xi)$ lässt sich einfach in obige Formel integrieren:

$$\sigma^2_{\text{mean}} = a \int d\beta d\alpha\, I^{-1}(\alpha) e^{p(\alpha,\beta)} k^2_{\text{mean}}(-R_{\text{F}}\sin\beta)\cos\beta.$$

Der Übergang zu Spiraldaten $p(\alpha,\beta,m)$ erfordert im Prinzip die genaue Berücksichtigung des z-Interpolationsalgorithmus. Dabei sind die Art des Algorithmus sowie die einstellbare resultierende effektive Schichtdicke $S_{\text{eff}}$ in die Fehlerfortpflanzung mit einzubeziehen. Allerdings hängt das Verhalten in Bezug auf den mittleren Rauschwert nicht vom Objekt ab, sondern kann in guter Näherung in die Proportionalitätskonstante $a$ gezogen werden. Damit ist $a$ nicht nur von der kollimierten Schichtdicke, sondern auch von weiteren Datenverarbeitungsschritten abhängig; eine einmalige, scannerabhängige Kalibrierung reicht jedoch aus.

Der Rauschwert im Bild an einer bestimmten z-Position $z_R = \alpha_R\, d\,/2\pi$ lässt sich nun durch Berücksichtigung des um $\alpha_R$ zentrierten 180° Teilscanintervalls analog zu obigen Gleichungen direkt aus den gemessenen Spiraldaten zu

$$\sigma^2_{\text{mean}}(\alpha_{\text{R}}) = a \int_{-\Phi/2}^{\Phi/2} d\beta \int_{\alpha_{\text{R}}-\pi/2}^{\alpha_{\text{R}}+\pi/2} d\alpha\, I^{-1}(\alpha) e^{p(\alpha,\beta,m)} k^2_{\text{mean}}(-R_{\text{F}}\sin\beta)\cos\beta \qquad (1)$$

errechnen. Die zur Auswertung verwendete gemessene Schicht $m$ ist frei wählbar, die in Vorschubrichtung vorne liegende Schicht (entweder $m = 1$ oder $m = M$) erscheint jedoch zur Rauschvorhersage am Besten geeignet. Die alternative Möglichkeit, eine Linearkombination aller $M$ gemessenen Schichten, ein Datenintervall von mehr als 180° oder gar die komplette z-Interpolation in obige Formel zu integrieren, soll hier durchaus offen gehalten werden. Es ist jedoch wahrscheinlich, dass dann die Vorhersagekraft der Methode durch die Tatsache, dass mehr als nur ein Halbumlauf an Informationen beitragen muss, schlechter ist als die der 180° Teilscan-Methode. Zur Evaluierung obiger Gleichung wird die Kenntnis des Röhrenstroms $I(\alpha)$ für die Winkel $\alpha \in (\alpha_R - \pi/2, \alpha_R + \pi/2)$ vorausgesetzt. Der aktuelle Röhrenstrom $I(\alpha_R + \pi/2)$ muss nun mit dem Faktor $\sigma^2_{\text{mean}}(\alpha_R)/\sigma^2_{\text{user}}$ oder einer davon abhängigen Funktion multipliziert werden um das Rauschniveau auf den vom Anwender gewünschten Wert $\sigma_{\text{User}}$ zu korrigieren.

Analoge Vorgehensweisen lassen sich auch für andere Rekonstruktionsmethoden ableiten. Beispielsweise wird oft eine direkte Fächerrekonstruktion ohne Rebinning auf Parallelkoordinaten durchgeführt [8]. Oder es wird eine direkte Fourierrekonstruktion (Gridding im Fourierraum) statt einer gefilterten Rückprojektion verwendet [9]. Für diese und andere Methoden zur Bildrekonstruktion aus Linienintegralen (z.B. CT-Daten) lassen sich äquivalente Rauschvorhersagen treffen, die hier aber nicht explizit aufgeführt werden sollen.

Für Rekonstruktionsalgorithmen, bei denen die Rauschvorhersage im Gegensatz zu obigen Erläuterungen keine derartige Vereinfachung im Vergleich zur kompletten Bildrekonstruktion erlaubt oder bei denen die Rauschvorhersage unter keinen Umständen in der erforderlichen Zeit (d.h. on-line) durchgeführt werden kann, bietet sich eine andere Möglichkeit. Hier kann zur Rauschvorhersage während der Messung, wie oben erläutert, eine einfache gefilterte Rückprojektion zu Grunde gelegt werden. Selbst wenn diese keine akzeptable Bildqualität liefert kann das durch Gleichung (1) vorhergesagte Rauschen unter Berücksichtigung des NPS (Noise Power Spectrum) des einfachen gefilterten Rückprojektionsalgorithmus und des NPS des komplizierten und rechenaufwendigen, aber qualitativ hochwertigen Rekonstruktionsalgorithmus bei der eigentlichen Bildrekonstruktion in den zu erwartenden Rauschwert umgerechnet werden. Diese Tatsache ist insbesondere für sogenannte Kegelstrahlscanner wichtig die so komplizierte Rekonstruktionsalgorithmen erfordern dass, die direkte Rauschvorhersage in der verfügbaren Zeit nicht möglich sein wird.

Methode 2, on-line Berechnung und Auswertung von Differenzbildern

Alternativ zu oben angegebener Methode kann der Umweg über den Bildraum gegangen werden. Dazu können aus den gemessenen Spiralrohdaten $p(\alpha,\beta,m)$ durch z-Interpolation und Rebinning auf Parallelkoordinaten zwei *unabhängige* Teilscandatensätze $p_1(\vartheta,\xi,z)$ und $p_2(\vartheta,\xi,z)$ erzeugt werden. Dabei darf kein gemessener Wert $p(\alpha,\beta,m)$ gleichzeitig zu beiden Teilscansinogrammen $p_1$ sowie $p_2$ beitragen. Dies kann erreicht werden in dem zum Erzeugen der Sinogramme die Symmetrie $(\vartheta,\xi) \leftrightarrow (\vartheta+\pi,-\xi)$ ausgenutzt wird. Rekonstruiert (z.B. mittels gefilterter Rückprojektion) wird nun das Differenzsinogramm $p(\vartheta,\xi,z)=p_1(\vartheta,\xi,z)-p_2(\vartheta,\xi,z)$, wobei ein Differenzbild entsteht aus dem die Standardabweichung $\sigma(z)$ des Pixelrauschens direkt berechnet werden kann. Der aktuelle Röhrenstrom $I(z)$ muss nun mit dem Faktor $\sigma^2(z)/\sigma^2_{user}$ oder einer davon abhängigen Funktion multipliziert werden, um das Rauschniveau auf den vom Anwender gewünschten Wert $\sigma_{user}$ zu korrigieren.

Um die notwendige Rekonstruktion zu beschleunigen und somit eine on-line Auswertung zu erlauben, besteht die Möglichkeit die Pixelanzahl, im rekonstruierten Bild sowie die Anzahl der zur Rekonstruktion beitragenden Projektionen (z.B. durch Verwendung von nur jeder $i$ - ten Projektion und/oder jedes $j$ -ten Detektors) zu reduzieren.

**Lösung Ziel 2: Modifizierte Röhrenstrommodulation (Dosisminimierung)**

Zur Rauschvorhersage und damit zur Vorhersage des zu verwendenden Röhrenstroms $I(\alpha)$ (bzw. äquivalent dazu $I(z)$, denn $z = d\alpha/2\pi$) wurden Daten aus einem 180° bzw. 360° langem Umlaufintervall benötigt. Die resultierende Funktion $I(\alpha)$ ensteht somit aus einer Mittelung über einen halben oder einen ganzen Umlauf des Strahlenfokus. Damit handelt es sich um eine relativ glatte Funktion die signifikante Änderungen nur auf einer 180° bzw. 360° Skala aufweist.

Die Röhrenstrommodulationstechniken hingegen gehen von einem konstanten Referenzstrom $I_0$ aus, auf den ein Projektionswinkelabhängiger Modulationsfaktor $\lambda(\alpha)$ aufmultipliziert wird um innerhalb eines 360° Umlaufs den Röhrenstrom an Projektionen geringer Schwächung (beim Patienten typischerweise a.p.) herabzusenken und an Projektionen hoher Schwächung (beim Patienten typischerweise lateral) anzuheben, mit dem Ziel die Varianz

$$\text{Var } P \approx a(I_0\lambda(\alpha))^{-1} \, e^p$$

über eine 360° Umlauf hinweg möglichst konstant zu halten ( P bezeichnet hier wieder die Z-Variable Messwert und $p$ eine zugehörige, gemessene Realisierung des Schwächungswerts, also ein Linienintegral durch das Objekt). Der Faktor $\lambda(\alpha)$ variiert innerhalb eines 180° Umlaufs signifikant wohingegen auf einer größeren Skala keine Änderungen vorhanden sind (d.h. eine gleitente Mittelung über 180° liefert im Wesentlichen eine konstante Funktion: $\lambda(\alpha) * II(\alpha/\pi) \approx const.$)

Damit eignet sich $I(\alpha)\lambda(\alpha)$ als optimaler Röhrenstrom der einerseits die Belichtungsautomatik (Ziel 1) in Form von $I(\alpha)$ und die Dosisminimierung durch Modulation in Form von $\lambda(\alpha)$ beinhaltet. Die on-line Bestimmung von $\lambda(\alpha)$ kann weiter wie beispielsweise in [3, 4] beschrieben stattfinden; aufgrund der unterschiedlichen Skalen von $I(\alpha)$ und $\lambda(\alpha)$ (langsame Schwankungen in der Belichtungsautomatik und schnelle Schwankungen der Modulation) ist die Unabhängigkeit und somit der Produktansatz $I(\alpha)\lambda(\alpha)$ gerechtfertigt.

**Lösung Ziel 3: Anpassung der 3D adaptiven Filterung**

Die adaptive Filterung besteht in einer schwellwertabhängigen lokalen Glättung der gemessenen Projektionswerte $p(\alpha,\beta,m)$ [6, 7]. Schwächungswerte, die einen bestimmten, projektionswinkelabhängigen Schwellwert $T(\alpha)$

überschreiten, werden unter Zuhilfenahme benachbarter Messwerte geglättet. Die Bestimmung der Funktion $T(\alpha)$, eventuell in Abhängigkeit des gemessenen Objekts, ist in der Literatur beschrieben und ist nicht Gegenstand dieses Patents [6, 7].

Im Allgemeinen wird $T(\alpha)$ durch Auswertung der gemessenen Daten $p(\alpha,\beta,m)$ berechnet, d.h. $T(\alpha') = F\{p(\alpha,\beta,m)\}(\alpha')$ ist ein Funktional von $p(\alpha,\beta,m)$. Bisherige Ansätze der adaptiven Filterung gingen davon aus, dass der Röhrenstrom $I_0$ während der gesamten Messung konstant bleibt. Die Varianz der Z-Variable $P_{\alpha,\beta,m}$ kann damit wie folgt abgeschätzt werden:

$$\text{Var } P_{\alpha,\beta,m} = a\, I_0^{-1}\, e^{p(\alpha,\beta,m)}.$$

Die Belichtungsautomatik und die Röhrenstrommodulation erzeugen allerdings folgende Situation:

$$\text{Var } P_{\alpha,\beta,m} = a(I(\alpha)\lambda(\alpha))^{-1} e^{p(\alpha,\beta,m)}.$$

Nach Umformung in

$$\text{Var } P_{\alpha,\beta,m} = a\, I_0^{-1}\, e^{p(\alpha,\beta,m)+\ln(I_0/I(\alpha)\lambda(\alpha))}$$

wird klar, dass sich die Schwellwertberechnung unter Berücksichtigung des zeitlichen Röhrenstromverlaufs wie folgt formulieren lässt:

$$T(\alpha')= F\{p(\alpha,\beta,m)+\ln(I_0/I(\alpha)\lambda(\alpha))\}(\alpha'). \qquad (2)$$

Mit anderen Worten: der Input des Schwellwertbestimmungsalgorithmus muss von $p(\alpha,\beta,m)$ *zu* $p(\alpha,\beta,m)+\ln(I_0/I(\alpha)\lambda(\alpha))$ abgewandelt werden. Die 3D adaptive Filterung selbst operiert unverändert auf $p(\alpha,\beta,m)$, nur die Schwellwertbestimmung verändert sich.

**Literatur:**

[1] Kalender W A, Seissler W, Klotz E, Vock P. Spiral volumetric CT with single-breathhold technique, continuous transport, and continuous scanner rotation. Radiology 1990; 176 (1): 181-183
[2] Kalender W A. Computertomographie, Publicis MCD, 2000
[3] Gies M, Kalender W A, Wolf H, Suess C, Madsen M T. Dose reduction in CT by anatomically adapted tube current modulation. I. Simulation studies. Med. Phys. 1999;26(11):2235-2247
[4] Kalender W A, Wolf H, Suess C. Dose reduction in CT by anatomically adapted tube current modulation. II. Phantom measurements. Med. Phys. 1999;26(11):2248-2253
[5] Kalender W A, Wolf H, Suess C, Gies M, Greess H, Bautz WA. Dose reduction in CT by on-line tube current control: Principles and validation on phantoms and cadavers. European Radiology 1999;9(2):323-328
[6] Kachelrieß M, Kalender W A. Computertomograph mit reduzierter Dosisbelastung bzw. reduziertem Bildpunktrauschen. Deutsches Patent- und Markenamt, 1999. Patent Specification DE 198 53 143
[7] Kachelrieß M, Kalender W A. Dose reduction by generalized 3D adaptive filtering for conventional and spiral single-, multirow and cone-beam CT: Theoretical considerations, simulations, phantom measurements and patient studies. Radiology 1999;233(P):283
[8] P Dreike, JW Goodman. Convolution reconstruction of fan beam projections. Computer Graphics and Image Processing 1976; 5:459-469
[9] H Schomberg, J Timmer. The gridding method for image reconstruction by Fourier transformation. IEEE Trans. Med. Imag. 1995;14(3):596-607

**EP 1 172 069 A1**

**Patentansprüche**

1. Computertomograph mit Ein- oder Mehrzeilendetektor, der über eine on-line Belichtungsautomatik durch optimale automatische Wahl des Röhrenstroms, sowie eine Methode zur Dosisminimierung durch on-line Röhrenstrommodulation und eine darauf abgestimmte Möglichkeit zur nachträglichen Rauschreduzierung verfügt.

2. Computertomograph nach Anspruch 1, bei dem die on-line Rauschvorhersage und damit die Belichtungsautomatik auf folgender Formel oder ähnlichen Varianten basieren:

$$\sigma_{\text{mean}}^2(\alpha_R) = a \int_{-\Phi/2}^{\Phi/2} d\beta \int_{\alpha_R-\pi/2}^{\alpha_R+\pi/2} d\alpha \, I^{-1}(\alpha) e^{p(\alpha,\beta,m)} k_{\text{mean}}^2 (-R_F \sin\beta)\cos\beta$$

3. Computertomograph nach Anspruch 1, bei dem zur on-line Rauschvorhersage Differenzbilder ausgewertet werden. Diese entstehen durch Rekonstruktion der Differenz zweier, unabhängiger, an der gleichen z-Position gültiger 180° Datensätze.

4. Computertomograph nach Anspruch 3, bei dem die genannte Teilscanrekonstruktion mit geringerer Auflösung als der Messgeometrie entsprechend durchgeführt werden kann um die Rechenzeit zu reduzieren.

5. Computertomograph nach Anspruch 2 und 3, bei dem zur Rauschvorhersage andere Rekonstruktionsalgorithmen angenommen werden als bei der späteren eigentlichen Bildrekonstruktion eingesetzt werden.

6. Computertomograph nach Anspruch 1, der eine an die Belichtungsautomatik oder andere Schwankungen des Röhrenstroms angepasste Röhrenstrommodulation erlaubt um die Patientendosis bei gleich bleibender Bildqualität zu minimieren.

7. Computertomograph nach Anspruch 1, der eine Nachverarbeitungsmöglichkeit mittels 3D adaptiver Filter enthält, die optimal auf den real zur Messung verwendeten Röhrenstromverlauf $I(\alpha)\lambda(\alpha)$ abgestimmt ist. Die Schwellwertbestimmung kann nach der Formel

$$T(\alpha') = F\{p(\alpha,\beta,m) + \ln(I_0/I(\alpha)\lambda(\alpha))\}(\alpha')$$

stattfinden.

**Figur 1: Geometrische Definitionen (axial).** $(\alpha, \beta)$ sind die unabhängigen Strahlkoordinaten in Fächerstrahlgeometrie, $(\vartheta, \xi)$ diejenigen in Parallelstrahlgeometrie. Der Winkel $\alpha$ bezeichnet den Drehwinkel des Fokus um das Rotationszentrum. Dieses definiert den Ursprung des $x - y -$ Koordinatensystems. Der Winkel $\beta$ eines einzelnen Strahls im Fächer mit Öffnungswinkel $\Phi$ (Fächerwinkel) liegt zwischen $\beta_1$ für den ersten Detektor und $\beta_M$ für den letzten Detektor. $\beta$ wird relativ zum Zentralstrahl gemessen und kann somit positives und negatives Vorzeichen annehmen. Auf gegebenem Strahl $(\beta, \alpha)$ steht die $\xi -$ Achse senkrecht. Die Parallelkoordinaten $(\vartheta, \xi)$ eines Strahls errechnen sich aus den Fächerkoordinaten $(\alpha, \beta)$ gemäß $\vartheta = \alpha + \beta$ und $\xi = R_F \sin \beta$, wobei mit $R_F$ der Abstand Fokus–Drehzentrum bezeichnet wird.

Europäisches
Patentamt

**EUROPÄISCHER RECHERCHENBERICHT**

Nummer der Anmeldung

EP 00 11 5267

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| D,X | DE 198 53 143 A (VAMP VERFAHREN UND APPARATE DE) 17. Juni 1999 (1999-06-17) * Seite 2, Zeile 45 - Zeile 65; Ansprüche 1,2 * | 1,6 | A61B6/03 G01N23/04 |
| A | | 7 | |
| | --- | | |
| D,X | KACHELRIESS M. ET AL.: "Dose reduction by generalized 3D adaptative filtering for conventional and spiral single- , multirow and cone-beam CT: Theoretical considerations, simulations, phantom measurements and patient studies." RADIOLOGY, 28. November 1999 (1999-11-28) - 3. Dezember 1999 (1999-12-03), Seite 283 XP000981379 usa * das ganze Dokument * | 1,6 | |
| | --- | | |
| D,A | KALENDAR W. ET AL.: "Dose reduction in CT by anatomically adapted tube current modulation." MEDICAL PHYSICS., Bd. 26, Nr. 11, - November 1999 (1999-11) Seiten 2248-2253, XP000979850 AMERICAN INSTITUTE OF PHYSICS. NEW YORK., US ISSN: 0094-2405 * Zusammenfassung * | 1 | RECHERCHIERTE SACHGEBIETE (Int.Cl.7) A61B G01N |
| | --- | | |
| | -/-- | | |

Der vorliegende Recherchenbericht wurde für alle Patentansprüche erstellt

| Recherchenort | Abschlußdatum der Recherche | Prüfer |
|---|---|---|
| DEN HAAG | 7. Februar 2001 | Hulne, S |

# EP 1 172 069 A1

Europäisches Patentamt

**EUROPÄISCHER RECHERCHENBERICHT**

Nummer der Anmeldung

EP 00 11 5267

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| D,A | KALENDAR W. ET AL.: "Dose reduction in CT by anatomically adapted tube current modulation." MEDICAL PHYSICS., Bd. 26, Nr. 11, - November 1999 (1999-11) Seiten 2235-2247, XP000979950 AMERICAN INSTITUTE OF PHYSICS. NEW YORK., US ISSN: 0094-2405 * Zusammenfassung * | 1 | |
| D,A | KALENDAR W.A.: "Dose reduction in CT by on-line tube current control: principles and validation on phantoms and cadavers." EUROPEAN RADIOLOGY, Bd. 9, - 1999 Seiten 3232-328, XP000981367 Germany * Zusammenfassung * | 1 | |

**RECHERCHIERTE SACHGEBIETE (Int.Cl.7)**

Der vorliegende Recherchenbericht wurde für alle Patentansprüche erstellt

| Recherchenort | Abschlußdatum der Recherche | Prüfer |
|---|---|---|
| DEN HAAG | 7. Februar 2001 | Hulne, S |

KATEGORIE DER GENANNTEN DOKUMENTE

X : von besonderer Bedeutung allein betrachtet
Y : von besonderer Bedeutung in Verbindung mit einer
anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
nach dem Anmeldedatum veröffentlicht worden ist
D : in der Anmeldung angeführtes Dokument
L : aus anderen Gründen angeführtes Dokument

& : Mitglied der gleichen Patentfamilie,übereinstimmendes
Dokument

EPO FORM 1503 03 82 (P04C03)

10

**ANHANG ZUM EUROPÄISCHEN RECHERCHENBERICHT
ÜBER DIE EUROPÄISCHE PATENTANMELDUNG NR.**

EP 00 11 5267

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

07-02-2001

| Im Recherchenbericht angeführtes Patentdokument | Datum der Veröffentlichung | Mitglied(er) der Patentfamilie | Datum der Veröffentlichung |
|---|---|---|---|
| DE 19853143 A | 17-06-1999 | KEINE | |

Für nähere Einzelheiten zu diesem Anhang : siehe Amtsblatt des Europäischen Patentamts, Nr. 12/82